(19) Europäisches Patentamt / European Patent Office / Office européen des brevets

(11) **EP 3 212 673 B1**

(12) **EUROPEAN PATENT SPECIFICATION**

(45) Date of publication and mention
of the grant of the patent:
**12.09.2018 Bulletin 2018/37**

(51) Int Cl.:
**C08B 13/00** (2006.01)   **C08L 1/32** (2006.01)
**C09D 101/32** (2006.01)   **A61K 47/38** (2006.01)

(21) Application number: **15791419.3**

(22) Date of filing: **21.10.2015**

(86) International application number:
**PCT/US2015/056586**

(87) International publication number:
**WO 2016/069338 (06.05.2016 Gazette 2016/18)**

(54) **PROCESS FOR PRODUCING A CELLULOSE ETHER ACETATE SUCCINATE**

VERFAHREN ZUR HERSTELLUNG EINES CELLULOSEETHER-ACETATSUCCINATS

PROCÉDÉ DE PRODUCTION D'ACÉTATE-SUCCINATE D'ÉTHER DE CELLULOSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.10.2014 US 201462073251 P**

(43) Date of publication of application:
**06.09.2017 Bulletin 2017/36**

(73) Proprietor: **Dow Global Technologies LLC
Midland, MI 48674 (US)**

(72) Inventors:
• **PETERMANN, Oliver
29699 Bomlitz (DE)**

• **SPREHE, Matthias
29699 Bomlitz (DE)**

(74) Representative: **f & e patent
Braunsberger Feld 29
51429 Bergisch Gladbach (DE)**

(56) References cited:
**WO-A1-2009/061815      WO-A1-2011/159626
WO-A1-2013/148154      WO-A1-2014/031448
WO-A1-2014/137779      WO-A1-2015/041973
US-A- 2 912 430           US-A- 3 497 496
US-A- 3 629 237**

**Description**

FIELD

The present invention relates to a process for preparing a cellulose ether acetate succinate.

INTRODUCTION

Cellulose ether acetate succinates, such as hydroxyalkyl methyl cellulose acetate succinates, their uses and processes for preparing them are generally known in the art. Various known hydroxyalkyl methyl cellulose acetate succinates are useful as enteric polymers for pharmaceutical dosage forms, such as hydroxypropyl methyl cellulose acetate succinate (HPMCAS). Enteric polymers are those that are resistant to dissolution in the acidic environment of the stomach. Dosage forms coated with such polymers protect the drug from inactivation or degradation in the acidic environment or prevent irritation of the stomach by the drug.

U.S. Patent No. 4,226,981 discloses a process for preparing mixed esters of cellulose ethers, such as hydroxypropyl methyl cellulose acetate succinate (HPMCAS), by esterifying hydroxypropyl methylcellulose with succinic anhydride and acetic anhydride in the presence of an alkali carboxylate as the esterification catalyst and acetic acid as the reaction medium.

US Patent No. 5,776,501 lists various cellulosic polymers, vinyl polymers or acrylic polymers as a basis for enteric coatings, such as cellulose acetate phthalate, cellulose acetate trimelliate, hydroxypropyl methylcellulose phthalate, hydroxypropyl methylcellulose acetate succinate and carboxymethylethyl cellulose, polyvinyl alcohol acetate phthalate, or copolymers of methacrylic acid and ethyl acrylate. More specifically, US Patent No. 5,776,501 teaches the usage of a water-soluble cellulose ether, such as hydroxypropyl methyl cellulose (HPMC), for producing hydroxypropyl methyl cellulose acetate maleate. The hydroxypropyl methyl cellulose acetate maleate is used as coating base for enteric pharmaceutical preparations. The water-soluble cellulose ether has a viscosity of 3 to 10 cp (= mPa·s), determined as a 2% by weight aqueous solution. If the viscosity is less than 3 cp, the finally obtained coating film for solid enteric pharmaceutical preparations is insufficient in strength, while if it exceeds 10 cp, the viscosity observed when the cellulose ether is dissolved in a solvent to carry out a substitution reaction becomes extremely high and homogeneous mixing and handling for further treatment is very difficult.

International patent applications WO 2005/115330 and WO 2011/159626 disclose the preparation of hydroxypropyl methylcellulose acetate succinate (HPMCAS). HPMC having an apparent viscosity of 2.4 to 3.6 cp is recommended as a starting material. Alternatively, a HPMC starting material of 600 to 60,000 Daltons, preferably 3000 to 50,000 Daltons, more preferably 6,000 to 30,000 Daltons is recommended. According to Keary [Keary, C.M.; Carbohydrate Polymers 45 (2001) 293-303, Tables 7 and 8] HPMC having a weight average molecular weight of about 85-100 kDa has a viscosity of about 50 mPa·s, determined as a 2% by weight aqueous solution. The produced HPMCAS is useful for enhancing the concentration of a dissolved active agent, for example for enhancing the bioavailability of poorly water-soluble drugs.

Unfortunately, the use of cellulose ethers having a viscosity in the range of 2.4 to 10 cp (= mPa·s) as starting material for the esterification, as recommended in the prior art, requires depolymerization of cellulose ethers to a large degree prior to the esterification process with acetic anhydride and succinic anhydride. Processes for depolymerizing cellulose ethers are described, for example, in European Patent Applications EP 1,141,029; EP 210,917; and EP 1,423,433; and in US Patent No. 4,316,982.

The known partial depolymerization of cellulose ethers with an acid, such as HCl, followed by neutralization before the esterification step results in a cost-intensive multistep production process.

Accordingly, it would be desirable to provide a process for preparing a cellulose ether acetate succinate, wherein the cellulose ether does not have to be depolymerized to a large degree before it is esterified with succinic anhydride and acetic anhydride.

SUMMARY

The present invention relates to a process for producing a cellulose ether acetate succinate which comprises the steps of reacting a cellulose ether with acetic anhydride, succinic anhydride and a depolymerizing agent, wherein the cellulose ether has an initial viscosity of at least 50 mPa·s, measured as a 2 weight-% solution in water at 20 °C, and the reaction of the cellulose ether with acetic anhydride, succinic anhydride and the depolymerizing agent is conducted simultaneously or in sequence in one reaction device.

DETAILED DESCRIPTION

The cellulose ether used as a starting material in the process of the present invention has an initial viscosity of at least 50 mPa·s, generally at least 80 mPa·s, preferably at least 150 mPa·s, more preferably at least 300 mPa·s or even at least 500 mPa·s, most preferably at least 1000 mPa·s or even at least 1500 mPa·s, and particularly at least 2000 mPa·s or even at least 3000 mPa·s, determined as a 2 weight-% solution in water at 20 °C. The cellulose ether generally has an initial viscosity of up to 100,000 mPa·s, typically up to 30,000 mPa·s, preferably up to 15,000 mPa·s, more preferably up to 7,000 mPa·s, and most preferably up to 5,000 mPa·s, determined as a 2 weight-% solution in water at 20 °C. The viscosity is determined as a 2 weight-% solution in water at 20 °C as described in the United States Pharmacopeia (USP 35, "Hypromellose", pages 423 - 424 and 3467 - 3469). As described in USP 35, viscosities of up to 600 mPa·s are determined by Ubbelohde viscosity measurement and viscosities above 600 mPa·s are determined using a Brookfield viscometer. Descriptions on preparing the 2 wt. % HPMC solution and both Ubbelohde and Brookfield viscosity measurement conditions are described USP 35.

The term "initial viscosity" as used herein means the viscosity of the cellulose ether before partial depolymerization of the cellulose backbone by the depolymerizing agent. When cellulose ethers of such a high viscosity are used as starting material for the reaction with succinic anhydride and acetic anhydride, a gel-like or dough-like inhomogeneous reaction mass results when the cellulose ether is combined with a diluent at a typical ratio of cellulose ether to diluent as described below. Particularly in one embodiment of the invention, where a cellulose ether is used as a starting material which has an initial viscosity of at least 500 mPa·s, measured as a 2 weight-% solution in water at 20 °C, a homogeneous reaction mixture cannot be obtained by stirring the reaction mass. Large lumps of starting materials like cellulose ethers are formed which neither dissolve nor are properly dispersible in the diluent. It is very surprising that an effective esterification with acetic anhydride and succinic anhydride and effective partial depolymerization with the depolymerizing agent can be achieved, without leaving a large amount of non-reacted or insufficiently reacted cellulose ether in spite of the physical properties of the reaction mass as described above.

U.S. Patent No. 3,629,237 discloses reacting phthalic anhydride with cellulose ether in the presence of an organic acid and a catalyst, such as anhydrous sodium acetate and potassium acetate, and in the presence of alkali metal salts of oxyacids of halogens, such as potassium chlorate, potassium bromated, and sodium chlorate, which facilitates the production of low-viscosity acid phthalates of cellulose ethers. Hydroxypropyl methylcellulose (HPMC) having a viscosity of 15 to 100 cps, determined as a 2% by weight aqueous solution, is used as a starting material for the reaction with phthalic anhydride.

It has surprisingly been found that that the use of a depolymerizing agent, typically an oxidizing agent such as potassium chlorate, is much more effective in the process for producing a cellulose ether acetate succinate than in the process for producing a cellulose ether phthalate. Significantly more molecular weight reduction is achieved in the production of cellulose ether acetate succinate than in the production of a comparable cellulose ether phthalate at comparable amounts of acid or a depolymerizing agent. Moreover, it has surprisingly been found that in the process of the present invention for producing cellulose ether acetate succinate it is even possible to use cellulose ethers of considerably higher initial viscosity than disclosed in U.S. Patent No. 3,629,237.

A depolymerizing agent is used in the esterification process of the present invention. In a preferred aspect of the invention the depolymerizing agent is selected from oxidizing agents, typically oxidizing agents which are different from oxygen, such as ozone, peroxides, halites, halates, perhalates, hypohalites and perborates, and hydrogen peroxide. Preferred depolymerizing agents are alkali metal chlorites, alkali metal chlorates, such as potassium chlorate or sodium chlorate, alkali metal perchlorates, alkali metal periodates, alkali metal hypobromites, alkali metal hypochlorites, alkali metal hypoiodites, alkali metal peroxides, and hydrogen peroxide. Sodium and potassium are the preferred alkali metals.

In another aspect of the invention the depolymerizing agent is an inorganic acid, such as a hydrogen halide, preferably hydrogen chloride; sulfuric acid, nitric acid, phosphoric acid, or persulfuric acid. When using an inorganic acid as depolymerizing agent, a neutralizing agent, such as sodium hydroxide, sodium bicarbonate, or sodium carbonate, is typically added to the reaction mixture after the desired degree of depolymerization has been achieved.

The amount of depolymerizing agent is generally at least 0.005 mole, preferably at least 0.01 mole, more preferably at least 0.02 mole, and most preferably at least 0.03 mole, per mole of anhydroglucose units of the cellulose ether. The amount of depolymerizing agent is generally up to 1 mole, preferably up to 0.70 mole, more preferably up to 0.40 mole, and most preferably up to 0.30 mole, per mole of anhydroglucose units of the cellulose ether.

One or more types of depolymerizing agents can be used, but their total amount is preferably chosen to be within the above described molar ratios between the depolymerizing agent and the anhydroglucose units of the cellulose ether.

The cellulose ether used as a starting material in the process of the present invention has a cellulose backbone having $\beta$-1,4 glycosidically bound D-glucopyranose repeating units, designated as anhydroglucose units in the context of this invention. The cellulose ether used as a starting material in the process of the present invention preferably is an alkyl cellulose, hydroxyalkyl cellulose or hydroxyalkyl alkylcellulose. This means that in the cellulose ether utilized in the process of the present invention, at least a part of the hydroxyl groups of the cellulose backbone of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred.

Illustrative of the above-defined cellulose ethers are alkylcelluloses, such as methylcellulose, ethylcellulose, and propylcellulose; hydroxyalkylcelluloses, such as hydroxyethylcellulose, hydroxypropylcellulose, and hydroxybutylcellulose; and hydroxyalkyl alkylcelluloses, such as hydroxyethyl methylcellulose, hydroxymethyl ethylcellulose, ethyl hydroxyethylcellulose, hydroxypropyl methylcellulose, hydroxypropyl ethylcellulose, hydroxybutyl methylcellulose, and hydroxybutyl ethylcellulose; and those having two or more hydroxyalkyl groups, such as hydroxyethylhydroxypropyl methylcellulose. Most preferably, the cellulose ether is a hydroxypropyl methylcellulose.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the cellulose ether. It is to be understood that during the hydroxyalkylation reaction the hydroxyl group of a hydroxyalkoxyl group bound to the cellulose backbone can be further etherified by an alkylation agent, e.g. a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxyl units are covalently bound to each other by ether bonding. Within this definition it is not important whether the terminal hydroxyl group of a hydroxyalkoxyl substituent is further alkylated or not; both alkylated and non-alkylated hydroxyalkoxyl substituents are included for the determination of MS(hydroxyalkoxyl). The esterified cellulose ether generally has a molar substitution of hydroxyalkoxyl groups of at least 0.05, preferably at least 0.08, more preferably at least 0.12, and most preferably at least 0.15. The degree of molar substitution is generally not more than 1.00, preferably not more than 0.90, more preferably not more than 0.70, and most preferably not more than 0.50.

The average number of hydroxyl groups substituted by alkoxyl groups, such as methoxyl groups, per anhydroglucose unit, is designated as the degree of substitution of alkoxyl groups, DS(alkoxyl). In the above-given definition of D S, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The cellulose ethers used as a starting material in the process of the present invention preferably have a DS(alkoxyl) of at least 1.0, more preferably at least 1.1, even more preferably at least 1.2, most preferably at least 1.4, and particularly at least 1.6. The DS(alkoxyl) is preferably not more than 2.5, more preferably not more than 2.4, even more preferably not more than 2.2, and most not more than 2.05. The degree of substitution of alkoxyl groups and the molar substitution of hydroxyalkoxyl groups can be determined by Zeisel cleavage of the cellulose ether with hydrogen iodide and subsequent quantitative gas chromatographic analysis (G. Bartelmus and R. Ketterer, Z. Anal. Chem., 286 (1977) 161-190). Most preferably the cellulose ether utilized in the process of the invention is hydroxypropyl methylcellulose having a DS(methoxyl) within the ranges indicated above for DS(alkoxyl) and an MS(hydroxypropoxyl) within the ranges indicated above for MS(hydroxyalkoxyl).

The cellulose ether is preferably used in its dried state as a starting material for producing the cellulose ether acetate succinate. "Dried state" means that the cellulose ether has a water content of not more than 5 percent, based on the weight of the moist cellulose ether, as defined in European Pharmacopeia Ph Eur 6 2008, Hypromellose, page 2819. Typically the water content is not more than 3 percent, based on the weight of the moist cellulose ether. The cellulose ether, preferably an above-mentioned hydroxypropyl methylcellulose, is used as a starting material to produce a cellulose ether acetate succinate, preferably hydroxypropyl methylcellulose acetate succinate (HPMCAS) as described below. The cellulose ether is reacted with acetic anhydride, succinic anhydride and a depolymerizing agent simultaneously or in sequence in one reaction device. Suitable reaction devices, such as batch reactors or reaction vessels, are known in the art. Preferred are reactors equipped with a stirring device or kneaders.

Preferred depolymerizing agents and preferred molar ratios between the depolymerizing agent and the anhydroglucose units of the cellulose ether are as described further above. Esterification of the cellulose ether with acetic acid and succinic anhydride can be partially or fully completed before the cellulose ether carrying additional ester groups is reacted with the depolymerizing agent. Alternatively, the cellulose ether can be first reacted with the depolymerizing agent and then with acetic acid and succinic anhydride. Even if the cellulose ether is reacted in sequence with acetic anhydride, succinic anhydride and a depolymerizing agent, the reactions take place in the same reaction device. Intermediate isolation steps or purification steps are not necessary and typically not carried out. Preferably, the reaction of the cellulose ether with acetic acid, succinic anhydride and a depolymerizing agent is conducted simultaneously. More preferably a reaction mixture is prepared which comprises the cellulose ether, acetic anhydride, succinic anhydride and the depolymerizing agent and the reaction mixture is subsequently heated to conduct the esterification reaction. The two anhydrides may be introduced into the reaction device, such as a known reaction vessel, at the same time or separately one after the other.

The amount of each anhydride to be introduced into the reaction device is determined depending on the desired degree of esterification to be obtained in the final product, usually being 1 to 10 times the stoichiometric amounts of the desired molar degree of substitution of the anhydroglucose units by esterification. The molar ratio between the acetic anhydride and the anhydroglucose units of the cellulose ether generally is 0.1 / 1 or more, preferably 0.3 / 1 or more, more preferably 0.5 / 1 or more, most preferably 1 / 1 or more, and particularly 1.5 / 1 or more. The molar ratio between the acetic anhydride and the anhydroglucose units of the cellulose ether generally is 17 / 1 or less, preferably 10 / 1 or less, more preferably 8 / 1 or less, most preferably 6 / 1 or less, and particularly 4 / 1 or less. The molar ratio between the succinic anhydride and the anhydroglucose units of cellulose ether preferably is 0.01 / 1 or more, more preferably 0.04 / 1 or more, and most preferably 0.2 / 1 or more. The molar ratio between the succinic anhydride and the anhydroglucose units of cellulose ether preferably is 2.5 / 1 or less, more preferably 1.5 / 1 or less, and most preferably 1 / 1 or less.

The molar number of anhydroglucose units of the cellulose ether utilized in the process of the present invention can be determined from the weight of the cellulose ether used as a starting material, by calculating the average molecular weight of the substituted anhydroglucose units from the DS(alkoxyl) and MS(hydroxyalkoxyl).

The esterification of the cellulose ether is typically conducted in a diluent, preferably in an aliphatic carboxylic acid as a reaction medium, such as acetic acid, propionic acid, or butyric acid. The reaction medium can comprise minor amounts of other solvents or diluents which are liquid at room temperature and do not react with the cellulose ether, such as aromatic or aliphatic solvents like benzene, toluene, 1,4-dioxane, or tetrahydrofurane; or halogenated $C_1$-$C_3$ derivatives, like dichloro methane or dichloro methyl ether, but the amount of the aliphatic carboxylic acid should generally be more than 50 percent, preferably at least 75 percent, and more preferably at least 90 percent, based on the total weight of the reaction medium. Most preferably the reaction medium consists of an aliphatic carboxylic acid. The esterification reaction is generally conducted in the presence of at least 200 parts, preferably at least 300 parts, more preferably at least 400 parts, and most preferably at least 600 parts by weight of the reaction medium, such as an aliphatic carboxylic acid, per 100 weight parts of the cellulose ether. The esterification reaction is generally conducted in the presence of up to 2000 parts, preferably up to 1500 parts, more preferably up to 1000 parts and most preferably up to 600 parts by weight of the reaction medium, such as an aliphatic carboxylic acid, per 100 weight parts of the cellulose ether.

The esterification reaction is generally conducted in the presence of an esterification catalyst, preferably in the presence of an alkali metal carboxylate, such as sodium acetate or potassium acetate. The amount of the alkali metal carboxylate is generally 20 to 200 parts by weight of the alkali metal carboxylate per 100 parts by weight of the cellulose ether. The molar ratio [alkali metal carboxylate / anhydroglucose units of cellulose ether] is preferably from [0.4 / 1.0] to [3.8 / 1.0], more preferably from [1.5 / 1.0] to [3.5/ 1.0], and most preferably from [1.9 / 1.0] to [3.0/ 1.0].

The reaction mixture is generally heated at 60 °C to 110 °C, preferably at 70 to 100 °C, for a period of time sufficient to complete the reaction, that is, typically from 2 to 25 hours, more typically from 2 to 8 hours.

The cellulose ether having an initial viscosity of at least 50 mPa·s, generally at least 80 mPa·s, preferably at least 150 mPa·s, more preferably at least 300 mPa·s or even at least 500 mPa·s, most preferably at least 1000 mPa·s or even at least 1500 mPa·s, and particularly at least 2000 mPa·s or even at least 3000 mPa·s, measured as a 2 weight-% solution in water at 20 °C, which is used as starting material in the process of the present invention has a very high thickening effect. As stated above, the viscosity is determined as a 2 weight-% solution in water at 20 °C as described in the United States Pharmacopeia (USP 35, "Hypromellose", pages 423 - 424 and 3467 - 3469).

When the cellulose ether has a viscosity of 500 mPa·s or more, and is combined with the aliphatic carboxylic acid at a weight ratio as disclosed above and with the other reactants as described above, a gel-like or dough-like inhomogeneous reaction mass results. A homogeneous reaction mixture cannot be obtained by stirring the reaction mass. Large lumps of starting materials like cellulose ethers are formed which neither dissolve nor are properly dispersible in the aliphatic carboxylic acid. Surprisingly, the inhomogeneous reaction mixture does not affect the quality of the produced esterified cellulose ether and the esterification reaction proceeds well. While the esterification and the partial depolymerization proceed, the viscosity of the reaction mixture decreases and its stirrability improves, but a homogeneous mixture generally can only be achieved after an extended time period, typically after 1 to 3 hours.

After completion of the esterification reaction, the reaction product can be precipitated from the reaction mixture in a known manner, for example by contacting with a large volume of water, such as described in U.S. Patent No. 4,226,981, International Patent Application WO 2005/115330 or European Patent Application EP 0 219 426. In a preferred embodiment of the invention the reaction product is precipitated from the reaction mixture as described in International Patent Application PCT/US13/030394, published as WO2013/148154, to produce an esterified cellulose ether in the form of a powder.

The cellulose ether acetate succinate produced according to the process of the present invention generally has a degree of substitution of acetyl groups of at least 0.05, preferably at least 0.10, more preferably at least 0.15, and most preferably at least 0.20. The degree of substitution of acetyl groups is generally up to 1.75, preferably up to 1.50, more preferably up to 1.25, and most preferably up to 1.00. The cellulose ether acetate succinate generally has a degree of substitution of succinoyl groups of at least 0.05, preferably at least 0.10. The degree of substitution of succinoyl groups is generally up to 1.6, preferably up to 1.30, more preferably up to 1.00, most preferably up to 0.70, and particularly up to 0.60. The sum of i) the degree of substitution of acetyl groups and ii) the degree of substitution of succinoyl groups is generally at least 0.10, preferably at least 0.20, more preferably at least 0.30, and most preferably at least 0.40. This sum is generally up to 1.9, preferably up to 1.55, more preferably up to 1.15, and particularly up to 1.00.

The content of the acetate and succinate ester groups is determined according to "Hypromellose Acetate Succinate, United States Pharmacopia and National Formulary, NF 29, pp. 1548-1550". Reported values are corrected for volatiles (determined as described in section "loss on drying" in the above HPMCAS monograph). The method may be used in analogue manner to determine the content of propionyl, butyryl, phthalyl and other ester groups.

The content of ether groups in the cellulose ether acetate succinate is determined in the same manner as described for "Hypromellose", United States Pharmacopeia and National Formulary, USP 35, pp 3467-3469.

The contents of ether and ester groups obtained by the above analyses are converted to DS and MS values of individual substituents according to the formulas below. The formulas may be used in analogue manner to determine the DS and MS of substituents of other cellulose ether esters.

$$
\begin{aligned}
\% \text{ cellulose backbone} \\
= 100 - \left( \%\text{MeO} * \frac{M(OCH_3) - M(OH)}{M(OCH_3)} \right) \\
- \left( \%\text{HPO} * \frac{M(OCH_2CH(OH)CH_3) - M(OH)}{M(OCH_2CH(OH)CH_3)} \right) \\
- \left( \%\text{Acetyl} * \frac{M(COCH_3) - M(H)}{M(COCH_3)} \right) \\
- \left( \%\text{Succinoyl} * \frac{M(COC_2H_4COOH) - M(H)}{M(COC_2H_4COOH)} \right)
\end{aligned}
$$

$$
DS(Me) = \frac{\dfrac{\%\text{MeO}}{M(OCH_3)}}{\dfrac{\%\text{cellulose backbone}}{M(AGU)}}
\qquad
MS(HP) = \frac{\dfrac{\%\text{HPO}}{M(HPO)}}{\dfrac{\%\text{cellulose backbone}}{M(AGU)}}
$$

$$
DS(Acetyl) = \frac{\dfrac{\%\text{Acetyl}}{M(Acetyl)}}{\dfrac{\%\text{cellulose backbone}}{M(AGU)}}
\qquad
DS(Succinoyl) = \frac{\dfrac{\%\text{Succinoyl}}{M(Succinoyl)}}{\dfrac{\%\text{cellulose backbone}}{M(AGU)}}
$$

M(MeO) = M(OCH$_3$) = 31.03 Da M(HPO) = M(OCH$_2$CH(OH)CH$_3$) = 75.09 Da M(Acetyl) = M(COCH$_3$) = 43.04 Da M(Succinoyl) = M(COC$_2$H$_4$COOH) = 101.08 Da M(AGU) = 162.14 Da M(OH) = 17.008 Da M(H) = 1.008 Da

By convention, the weight percent is an average weight percentage based on the total weight of the cellulose repeat unit, including all substituents. The content of the methoxyl group is reported based on the mass of the methoxyl group (i.e., -OCH$_3$). The content of the hydroxyalkoxyl group is reported based on the mass of the hydroxyalkoxyl group (i.e., -O-alkylene-OH); such as hydroxypropoxyl (i.e., -O-CH$_2$CH(CH$_3$)-OH). The content of acetyl group is reported based on the mass of - C(O)-CH$_3$. The content of the succinoyl groups is reported based on the mass of this group, i.e., - C(O) - CH$_2$ - CH$_2$ - COOH.

The cellulose ether acetate succinates produced according to the process of the present invention generally have a weight average molecular weight M$_w$ of 10,000 Dalton or more, preferably 20,000 Dalton or more, more preferably 30,000 Dalton or more, and most preferably 80,000 Dalton or more. The cellulose ether acetate succinates generally have a weight average molecular weight M$_w$ of up to 500,000 Dalton, preferably up to 450,000 Dalton, more preferably up to 350,000 Dalton, even more preferably up to 250,000 Dalton, and particularly up to 200,000 Dalton or up to 120,000 Dalton. The weight average molecular weight M$_w$ of the cellulose ether acetate succinate is typically at least 25 percent lower, more typically at least 40 percent lower than the weight average molecular weight of the cellulose ether used as a starting material for producing the cellulose ether acetate succinate, which reduction in $M_w$ is caused by the treatment with the depolymerizing agent.

The cellulose ether acetate succinates typically have a Polydispersity $M_w/M_n$ of at least 1.3, and more typically at least 1.5. Moreover, the esterified cellulose ethers of the present invention typically have a Polydispersity of up to 3.5, preferably up to 3.0, more preferably up to 2.8, and most preferably of up to 2.6. The Polydispersity $M_w/M_n$ is calculated based on the determination of the weight average molecular weight $M_w$ and the number average molecular weight $M_n$.

The cellulose ether acetate succinates generally have a number average molecular weight $M_n$ of 5000 Dalton or more, preferably 10,000 Dalton or more, more preferably 20,000 Dalton or more, and most preferably 25,000 Dalton or more. The esterified cellulose ethers of the present invention generally have a number average molecular weight $M_n$ of up to 150,000 Dalton, preferably up to 110,000 Dalton, more preferably up to 90,000 Dalton, and most preferably up to 50,000 Dalton.

$M_w$ and $M_n$ are measured by SEC-MALLS using as mobile phase a mixture which has been produced by mixing 40 parts by volume of acetonitrile and 60 parts by volume of aqueous buffer containing 50 mM $NaH_2PO_4$ and 0.1 M $NaNO_3$. The mobile phase is adjusted to a pH of 8.0. SEC-MALLS stands for Size Exclusion Chromatography coupled with a mass sensitive Multi Angle Laser Light Scattering detector. The procedure is described in Journal of Pharmaceutical and Biomedical Analysis 56 (2011) 743-748. The measurement of $M_w$ and $M_n$ is described in more details in the Examples.

The cellulose ether acetate succinates generally have a viscosity of up to 200 mPa·s, preferably up to 100 mPa·s, more preferably up to 50 mPa·s, even more preferably up to 30 mPa·s, most preferably up to 10 mPa·s, and particularly up to 5 mPa·s, measured as a 2.0 wt.-% solution of the esterified cellulose ether in 0.43 wt.-% aqueous NaOH at 20 °C. Generally the viscosity is at least 1.2 mPa·s, typically at least 1.8 mPa·s, and more typically at least 2.4 mPa·s, measured as a 2.0 wt.-% solution of the esterified cellulose ether in 0.43 wt.-% aqueous NaOH at 20 °C. The 2.0 % by weight solution of the esterified cellulose ether is prepared as described in"Hypromellose Acetate Succinate, United States Pharmacopia and National Formulary, NF 29, pp. 1548-1550", followed by an Ubbelohde viscosity measurement according to DIN 51562-1:1999-01 (January 1999).

Some embodiments of the invention will now be described in detail in the following Examples.

EXAMPLES

Unless otherwise mentioned, all parts and percentages are by weight. In the Examples the following test procedures are used.

Viscosity of Hydroxypropyl Methyl Cellulose (HPMC) samples

The viscosity of the cellulose ether was determined as a 2 weight-% solution in water at 20 °C as described in the United States Pharmacopeia (USP 35, "Hypromellose", pages 423 - 424 and 3467 - 3469). As described in the United States Pharmacopeia, viscosities of less than 600 mPa·s were determined by an Ubbelohde viscosity measurement and viscosities of 600 mPa·s or more were determined using a Brookfield viscometer. Descriptions on preparing the 2 wt. % HPMC solution and both Ubbelohde and Brookfield viscosity measurement conditions are disclosed in the United States Pharmacopeia (USP 35, "Hypromellose", pages 423 - 424 and 3467 - 3469 and in ASTM D-445 and ISO 3105 referenced therein).

Viscosity of Hydroxypropyl Methyl Cellulose Acetate Succinate (HPMCAS)

The 2.0 % by weight solution of the HPMCAS in 0.43 wt.-% aqueous NaOH was prepared as described in"Hypromellose Acetate Succinate, United States Pharmacopia and National Formulary, NF 29, pp. 1548-1550", followed by an Ubbelohde viscosity measurement at 20 °C according to DIN 51562-1:1999-01 (January 1999).

Content of ether and ester groups of HPMCAS

The content of ether groups in the HPMCAS was determined in the same manner as described for "Hypromellose", United States Pharmacopeia and National Formulary, USP 35, pp 3467-3469.

The ester substitution with acetyl groups ($-CO-CH_3$) and the ester substitution with succinoyl groups ($-CO-CH_2-CH_2-COOH$) were determined according to Hypromellose Acetate Succinate, United States Pharmacopia and National Formulary, NF 29, pp. 1548-1550". Reported values for ester substitution were corrected for volatiles (determined as described in section "loss on drying" in the above HPMCAS monograph).

Determination of $M_w$ and $M_n$ of HPMCAS

Mw and Mn were measured according to Journal of Pharmaceutical and Biomedical Analysis 56 (2011) 743 unless stated otherwise. The mobile phase was a mixture of 40 parts by volume of acetonitrile and 60 parts by volume of aqueous buffer containing 50 mM $NaH_2PO_4$ and 0.1 M $NaNO_3$. The mobile phase was adjusted to a pH of 8.0. Solutions of the cellulose ether esters were filtered into a HPLC vial through a syringe filter of 0.45 $\mu$m pore size.

More specifically, the utilized Chemicals and solvents were:

Polyethylene oxide standard materials (abbreviated as PEOX 20 K and PEOX 30 K) were purchased from Agilent Technologies, Inc. Palo Alto, CA, catalog number PL2083-1005 and PL2083-2005.

Acetonitrile (HPLC grade $\geq$ 99.9 %, CHROMASOL plus), catalog number 34998, sodium hydroxide (semiconductor grade, 99.99 %, trace metal base), catalog number 306576, water (HPLC grade, CHROMASOLV Plus) catalog number 34877 and sodium nitrate (99,995 %, trace metal base) catalog number 229938 were purchased from Sigma-Aldrich, Switzerland.

Sodium dihydrogen phosphate ($\geq$ 99.999 % TraceSelect) catalog number 71492 was purchased from FLUKA, Switzerland.

The normalization solution of PEOX20 K at 5 mg/mL, the standard solution of PEOX30 K at 2 mg/mL, and the sample solution of HPMCAS at 2 mg/mL were prepared by adding a weighed amount of polymer into a vial and dissolving it with a measured volume of mobile phase. All solutions were allowed to dissolve at room temperature in the capped vial for 24 h with stirring using a PTFE-coated magnetic stirring bar.

The normalization solution (PEOX 20k, single preparation, N) and the standard solution (PEOX30 K, double preparation, S1 and S2) were filtered into a HPLC vial through a syringe filter of 0.02 $\mu$m pore size and 25 mm diameter (Whatman Anatop 25, catalog number 6809-2002), Whatman.

The test sample solution (HPMCAS, prepared in duplicate, T1, T2) and a laboratory standard (HPMCAS, single preparation, LS) were filtered into a HPLC vial through a syringe filter of 0.45 $\mu$m pore size (Nylon, e.g. Acrodisc 13 mm VWR catalog number 514-4010).

Chromatographic condition and run sequence were conducted as described by Chen, R. et al.; Journal of Pharmaceutical and Biomedical Analysis 56 (2011) 743- 748). The SEC-MALLS instrument set-up included a HP1100 HPLC system from Agilent Technologies, Inc. Palo Alto, CA; a DAWN Heleos II 18 angle laser light scattering detector and a OPTILAB rex refractive index detector, both from Wyatt Technologies, Inc. Santa Barbara, CA. The analytical size exclusion column (TSK-GEL® GMPWXL, 300 $\times$ 7.8 mm) was purchased from Tosoh Bioscience. Both the OPTILAB and the DAWN were operated at 35 °C. The analytical SEC column was operated at room temperature (24 $\pm$ 5 °C). The mobile phase was a mixture of 40 volume parts of acetonitrile and 60 volume parts of aqueous buffer containing 50 mM NaH2PO4 and 0.1 M NaNO3 prepared as follows:

Aqueous buffer: 7.20 g of sodium dihydrogen phosphate and 10.2 g of sodium nitrate were added to 1.2 L purified water in a clean 2 L glass bottle under stirring until dissolution.

Mobile phase: 800 mL of acetonitrile were added to 1.2 L of the aqueous buffer prepared above, and stirred until a good mixture was achieved and the temperature equilibrated to ambient temperature.

The mobile phase was pH adjusted to 8.0 with 10M NaOH and filtered through a 0.2 m nylon membrane filter. The flow rate was 0.5 mL/min with in-line degassing. The injection volume was 100 $\mu$L and the analysis time was 35 min.

The MALLS data were collected and processed by Wyatt ASTRA software (version 5.3.4.20) using dn/dc value (refractive index increment) of 0.120 mL/g for HPMCAS. The light scattering signals of detector Nos. 1-4, 17, and 18) were not used in the molecular weight calculation. A representative chromatographic run sequence is given below: B, N, LS, S1 (5x), S2, T1 (2x), T2 (2x), T3 (2x), T4 (2x), S2, T5(2x), etc., S2, LS, W, where, B represents blank injection of mobile phase, N1 represents normalization solution; LS represents a laboratory standard HPMCAS; S1 and S2 represent standard solutions one and two, respectively; T1, T2, T3, T4, and T5 represent test sample solutions and W represents water injection. (2x) and (5x) denote the number of injections of the same solution.

Both the OPTILAB and the DAWN were calibrated periodically according to the manufacturer's recommended procedures and frequency. A 100 $\mu$L injection of a 5 mg/mL polyethylene oxide standard (PEOX20 K) was employed for normalizing all angle light scattering detectors relative to 90° detector for each run sequence.

Use of this mono-dispersed polymer standard also enabled the volume delay between the OPTILAB and the DAWN to be determined, permitting proper alignment of the light scattering signals to the refractive index signal. This is necessary for the calculation of the weight-averaged molecular weight (Mw) for each data slice.

Utilized Hydroxypropyl methylcellulose (HPMC)

The HPMC E3 LV had a methoxyl substitution ($DS_M$) and hydroxypropoxyl substitution ($MS_{HP}$) as listed in Table 2 below and a viscosity of 3.2 mPa·s, measured as a 2 wt.-% solution in water at 20 °C according to the Ubbelohde method described above. The weight average molecular weight of the HPMC E3 LV was about 20,000 Dalton. The HPMC E3 LV is a low viscosity HPMC which is commercially available from The Dow Chemical Company as Methocel E3 LV Premium cellulose ether. It is produced by partial depolymerization of higher molecular weight HPMC.

The HPMC E4M had a methoxyl substitution ($DS_M$) and hydroxypropoxyl substitution ($MS_{HP}$) as listed in Table 2 below and a viscosity of 3729 mPa·s, measured as a 2 % solution in water at 20 °C according to the Brookfield method described above using Rotor No. 4 at 60 rpm. The weight average molecular weight of the HPMC E4M was 305330 Dalton (305 kDa). The HPMC E4M is a high viscosity HPMC which is commercially available from The Dow Chemical Company as Methocel E4M cellulose ether.

The HPMC E100 LV contained 29.6 % methoxyl groups and 9.6 % hydroxypropoxyl groups, which corresponds to a methoxyl substitution ($DS_M$) of 1.95 and a hydroxypropoxyl substitution ($MS_{HP}$) of 0.26, and a viscosity of 87 mPa·s, measured as a 2 % solution in water at 20 °C according to the Ubbelohde method described above The number average molecular weight of the HPMC E100 LV was 74,000 Dalton. The weight average molecular weight of the HPMC E100 LV was about 120,000 Dalton. Methocel HPMC E100 LV was obtained by partial depolymerization of Methocel E4M Premium cellulose ether.

Production of HPMCAS of Examples 1 - 8

Glacial acetic acid, HPMC E4M, acetic anhydride, succinic anhydride, sodium acetate (water free) and potassium chlorate were introduced in the amounts listed in Table 1 below into a reaction vessel of 3L volume. The glacial acetic acid was readily absorbed by the HPMC powder. The mass in the reaction vessel had a solid appearance. The mass in the reaction vessel was heated at 85° C for 3.5 hours to effect esterification. During the heating period the mass formed a large lump and could not be stirred. At 85° C the mass could be stirred but no homogeneous mixture could be achieved. While the esterification and the partial depolymerization proceeded, the stirring ability of reaction mixture improved, but a homogeneous mixture could still not be achieved for an extended time period. After the reaction had proceeded for 1 hour, the reaction mass still displayed inhomogeneous lumps. After the completion of the reaction after 3.5 hours a homogeneous reaction solution was formed as during the reaction in Comparative Example A. Then 1.8 L of cold water was added to the reaction mixture under stirring to precipitate the HPMCAS. The precipitated product was removed from the reactor and washed with 20 L of water by applying high shear mixing using an Ultra-Turrax stirrer S50-G45 running at 5200 rpm. The product was isolated by filtration and dried at 55°C overnight.

In Example 3 the amount of acetic acid was increased to evaluate whether the stirrability of the reaction mass would be improved by an increased amount of acetic acid. However, no significant improvement was detectable.

Production of HPMCAS of Comparative Example A

The procedure of Examples 1 - 8 was repeated, except that low viscosity HPMC E3 LV was used instead of high viscosity HPMC E4M and the reaction was carried out in the absence of potassium chlorate. The reaction mixture consisting of glacial acetic acid, HPMC E3 LV, acetic anhydride, succinic anhydride and sodium acetate (water free) as listed in Table 1 below was well stirrable from the start.

The properties of the produced HPMCAS are listed in Table 2 below. All HPMCAS products of Examples 1 - 8 were white to off-white powders. The comparison of the HPMCAS produced according to Examples 1 - 8 and Comparative Example A illustrates the surprising fact that essentially the same degree of esterification is achieved using the same amount of esterification agents regardless whether a low viscosity cellulose ether is used as a starting material as in Comparative Example A or a high viscosity cellulose ether as in Examples 1 - 8. Also, essentially the same or an even lower polydispersity, $M_w/M_n$, is achieved in Examples 1 - 4 as in Comparative Example A. The $M_w$ and $M_n$ of the HPMCAS of Comparative Example A are within the ranges of $M_w$ and $M_n$ for Examples 1 - 4. The viscosity, determined as a 2.0 wt.-% solution in 0.43 wt. % aqueous NaOH, of the HPMCAS of Comparative Example A is even slightly higher than the viscosity of the HPMCAS of Examples 1 - 4. Essentially the same or an even lower polydispersity in Examples 1 - 4 means a comparable or even a more homogeneous distribution of the lengths of the polymer chains in Examples 1 - 4 than in Comparative Example A. This is highly surprising considering that no homogeneous reaction mixture could be achieved in Examples 1 - 8 by stirring, as explained in greater details above. The present invention avoids the known partial depolymerization of cellulose ethers with an acid, such as HCl, followed by neutralization before the esterification step. Hence the cost-intensive multistep production process of the prior art is simplified and the number of reaction steps is reduced. Thus the production costs are significantly reduced while the same quality of the cellulose ether acetate succinate is achieved. Moreover, controlling and varying the amount of depolymerizing agent utilized in the process of the present invention is a simple means for controlling and varying the number average molecular weight $M_n$ and the weight average molecular weight $M_w$ of the produced cellulose ether acetate succinate.

Table 1

| (Comp.) Example | Type HPMC used as starting material | Amount HPMC* | | acetic acid | | Succinic anhydride | | Acetic anhydride | | Sodium acetate | | Potassium chlorate | |
|---|---|---|---|---|---|---|---|---|---|---|---|---|---|
| | | g | mol | g | mol/mol HPMC | g | mol/mol HPMC | g | mol/mol HPMC | g | mol/mol HPMC | g | mol/mol HPMC |
| A | HPMC E3 LV | 150 | 0.74 | 750 | 16.8 | 42.0 | 0.57 | 195.0 | 2.69 | 150.0 | 2.47 | -- | -- |
| 1 | HPMC E4M | 150 | 0.74 | 750 | 16.8 | 42.0 | 0.57 | 195.0 | 2.69 | 150.0 | 2.47 | 20 | 0.22 |
| 2 | HPMC E4M | 150 | 0.74 | 750 | 16.8 | 42.0 | 0.57 | 195.0 | 2.69 | 150.0 | 2.47 | 15 | 0.165 |
| 3 | HPMC E4M | 150 | 0.74 | 750 + 320 | | 42.0 | 0.57 | 195.0 | 2.69 | 150.0 | 2.47 | 10 | 0.11 |
| 4 | HPMC E4M | 150 | 0.74 | 750 | 16.8 | 42.0 | 0.57 | 195.0 | 2.69 | 150.0 | 2.47 | 7.5 | 0.083 |
| 5 | HPMC E4M | 150 | 0.74 | 750 | 16.8 | 42.0 | 0.57 | 195.0 | 2.69 | 150.0 | 2.47 | 4 | 0.044 |
| 6 | HPMC E4M | 150 | 0.74 | 750 | 16.8 | 42.0 | 0.57 | 195.0 | 2.69 | 150.0 | 2.47 | 3 | 0.033 |
| 7 | HPMC E4M | 150 | 0.74 | 750 | 16.8 | 42.0 | 0.57 | 195.0 | 2.69 | 150.0 | 2.47 | 2 | 0.022 |
| 8 | HPMC E4M | 150 | 0.74 | 750 | 16.8 | 42.0 | 0.57 | 195.0 | 2.69 | 150.0 | 2.47 | 1 | 0.011 |
| * calculated on the dried basis | | | | | | | | | | | | | |

Table 2

| (Comp.) Example | Molecular weight (kDa) | | | Ether Substitution | | Ester substitution | | Ether Substitution | | Ester substitution | | 2% viscosity in NaOH [mPa·s] |
|---|---|---|---|---|---|---|---|---|---|---|---|---|
| | Mn | Mw | Mw/ Mn | Methoxyl (%) | Hydroxy-propoxyl (%) | Acetyl (%) | Succinoyl (%) | $DS_M$ | $MS_{HP}$ | $DOS_{Ac}$ | $DOS_s$ | |
| A | 23 | 44 | 1.9 | 23.6 | 7.7 | 8.8 | 11.8 | 1.95 | 0.26 | 0.53 | 0.30 | 2.93 |
| 1 | 15 | 27 | 1.8 | 21.5 | 7.2 | 10.0 | 11.9 | 1.78 | 0.25 | 0.60 | 0.30 | 1.66 |
| 2 | 17 | 31 | 1.8 | 21.9 | 7.3 | 9.3 | 11.6 | 1.79 | 0.25 | 0.55 | 0.29 | 1.79 |
| 3 | 24 | 37 | 1.5 | 22.5 | 7.6 | 8.0 | 11.1 | 1.80 | 0.25 | 0.46 | 0.27 | 2.37 |
| 4 | 29 | 53 | 1.8 | 22.2 | 7.4 | 8.9 | 11.9 | 1.82 | 0.25 | 0.53 | 0.30 | 2.39 |
| 5 | 37 | 81 | 2.2 | 23.0 | 7.5 | 8.7 | 11.6 | 1.88 | 0.25 | 0.51 | 0.29 | 3.52 |
| 6 | 43 | 101 | 2.3 | 23.0 | 7.5 | 8.9 | 11.5 | 1.89 | 0.25 | 0.53 | 0.29 | 4.38 |
| 7 | 50 | 138 | 2.8 | 23.2 | 7.5 | 8.7 | 11.5 | 1.90 | 0.25 | 0.51 | 0.29 | 5.68 |
| 8 | 83 | 253 | 3.0 | 23.1 | 7.6 | 8.7 | 11.7 | 1.9 | 0.26 | 0.52 | 0.30 | 10.2 |

Production of HPMCAS of Examples 9 and 10

Glacial acetic acid, acetic anhydride, succinic anhydride, HPMC E100 LV, sodium acetate (water free) and potassium chlorate were introduced in the amounts listed in Table 3 below into a reaction vessel of 1L volume. The weight ratios of the reactants in Examples 9 and 10 correspond to those in Example 1-5 of U.S. Patent No. 4,226,981 (Table 1), except that no potassium chlorate was used in the procedure of U.S. Patent No. 4,226,981.

The glacial acetic acid was readily absorbed by the HPMC powder. The reaction mixture had a solid appearance and could not be sufficiently stirred to achieve a homogeneous reaction mixture. The mixture was heated at 80° C for 5 hours to effect esterification. While the esterification and the partial depolymerization proceeded, the viscosity of the reaction mixture decreased and the reaction mixture could be sufficient stirred to provide a homogeneous mixture. After 5 hours the HPMCAS was precipitated from the reaction mixture by addition of 800 mL of water and cooling to room temperature. The precipitated product was removed from the reactor and washed with 1.9 L of water by applying high shear mixing using an Ultra-Turrax stirrer S50-G45. The product was isolated by filtration and dried at 60°C overnight.

Production of Hydroxypropyl Methyl Cellulose Phthalate (HPMCP) of Comparative Examples B and C

Glacial acetic acid, phthalic anhydride, sodium acetate (water free) and potassium chlorate were introduced in the amounts listed in Table 3 below into a reaction vessel of 1L volume. The weight ratios of the reactants in Comparative Example B correspond to those in Example 2-3 (Table 3) of U.S. Patent No. 3,629,237.

Table 3

| (Comp.) Example | HPMC | Amount HPMC | | acetic acid | | Phthalic anhydride | | Succinic anhydride | | Acetic anhydride | | Sodium acetate | | Potassium chlorate | |
|---|---|---|---|---|---|---|---|---|---|---|---|---|---|---|---|
| | | g | mol | g | mol/mol HPMC | g | mol/mol HPMC | g | mol/mol HPMC | g | mol/mol HPMC | g | mol/mol HPMC | g | mol/mol HPMC |
| B | E100 LV | 75 | 0.37 | 300 | 13.4 | 90 | 1.64 | -- | -- | -- | -- | 75 | 2.47 | 3.75 | 0.083 |
| 9 | E100 LV | 75 | 0.37 | 375 | 16.8 | -- | -- | 22.5 | | 120 | 3.3 | 75 | 2.47 | 3.75 | 0.083 |
| C | E100 LV | 75 | 0.37 | 300 | 13.4 | 90 | 1.64 | -- | | -- | -- | 75 | 2.47 | 2.51 | 0.055 |
| 10 | E100 LV | 75 | 0.37 | 375 | 16.8 | -- | -- | 22.5 | 0.61 | 120 | 3.3 | 75 | 2.47 | 2.51 | 0.055 |

Table 4

| (Comp.) Example | Molecular weight (kDA) | | 2% viscosity in NaOH (mPa·s) | Ether Substitution | | Ester substitution | | | Ether Substitution | | Ester substitution | | |
|---|---|---|---|---|---|---|---|---|---|---|---|---|---|
| | Mn | Mw | | Methoxyl (%) | Hydroxy-propoxyl (%) | Acetyl (%) | Phthalyl (%) | Succinoyl (%) | $DS_M$ | $MS_{HP}$ | $DOS_{Ac}$ | $DOS_P$ | $DOS_s$ |
| B | 47 | 74 | 4.1 | 18.8 | 6.3 | -- | 34.4 | -- | 1.87 | 0.26 | -- | 0.71 | -- |
| 9 | 24 | 47 | 2.4 | 23.0 | 7.4 | 9.3 | -- | 11.6 | 1.90 | 0.25 | 0.55 | -- | 0.29 |
| C | 57 | 87 | 5.0 | 18.7 | 6.2 | -- | 33.7 | -- | 1.83 | 0.25 | -- | 0.69 | -- |
| 10 | 26 | 53 | 2.7 | 23.4 | 7.5 | 9.7 | -- | 12.1 | 1.97 | 0.26 | 0.59 | -- | 0.31 |

The comparison between Comparative Example B and Example 9 and the comparison between Comparative Example C and Example 10 illustrate that the use of a depolymerizing agent, such as potassium chlorate, is much more effective in the process for producing a cellulose ether acetate succinate than in the process for producing a cellulose ether phthalate. Significantly more molecular weight reduction and viscosity reduction is achieved in the production of HPMCAS than in the production of hydroxypropyl methyl phthalate at comparable amounts of potassium chlorate. The phthalyl content does not cause the much higher number average molecular weight $M_n$ and weight average molecular weight $M_w$ in Comparative Examples B and C than in Examples 9 and 10.

The production of hydroxypropyl methyl cellulose acetate succinate (HPMCAS) is not directly comparable to the production of hydroxypropyl methyl cellulose phthalate (HPMCP) because in the latter process only one esterifying agent is used. However, to allow a comparison which is as fair as possible, in Examples 9 and 10 the weight ratios between HPMC, acetic acid, acetic anhydride and phthalic anhydride were chosen as recommended in the prior art (Example 1-5 of U.S. Patent No. 4,226,981 (Table 1)) and the weight ratio between potassium chlorate and HPMC, which is relevant for the depolymerization, was chosen exactly as disclosed in U.S. Patent No. 3,629,237.

**Claims**

1. A process for producing a cellulose ether acetate succinate comprising the step of reacting a cellulose ether with acetic anhydride and succinic anhydride in the presence of an aliphatic carboxylic acid and with a depolymerizing agent, wherein the cellulose ether has an initial viscosity of at least 50 mPa·s, measured as a 2 weight-% solution in water at 20 °C, the depolymerizing agent is an inorganic acid or an oxidizing agent different from oxygen and the reaction of the cellulose ether with acetic anhydride, succinic anhydride and the depolymerizing agent is conducted simultaneously or in sequence in one reaction device.

2. The process of claim 1 wherein the cellulose ether has a viscosity of at least 500 mPa·s, measured as a 2 weight-% solution in water at 20 °C.

3. The process of claim 1 wherein the cellulose ether has a viscosity of at least 1000 mPa·s, measured as a 2 weight-% solution in water at 20 °C.

4. The process of any one of claims 1 to 3 wherein a reaction mixture comprising the cellulose ether, acetic anhydride, succinic anhydride and the depolymerizing agent is prepared and the reaction mixture is subsequently heated to conduct the esterification reaction.

5. The process of any one of claims 1 to 4 wherein the amount of the depolymerizing agent is at least 0.03 mole per mole of anhydroglucose units of the cellulose ether.

6. The process of any one of claims 1 to 5 wherein the depolymerizing agent is selected from the group consisting of alkali metal chlorites, alkali metal chlorates, alkali metal perchlorates, alkali metal periodates, alkali metal hypobromites, alkali metal hypochlorites, alkali metal hypoiodites, alkali metal peroxides, and hydrogen peroxide.

7. The process of claim 6 wherein the depolymerizing agent is potassium chlorate or sodium chlorate.

8. The process of any one of claims 1 to 7 wherein the cellulose ether is alkyl cellulose, hydroxyalkylcellulose or hydroxyalkylalkylcellulose.

9. The process of claim 8 wherein hydroxypropyl methylcellulose is esterified with succinic anhydride and acetic anhydride to produce hydroxypropyl methyl cellulose acetate succinate.

10. The process of any one of claims 1 to 9 wherein the weight average molecular weight of the cellulose ether acetate succinate is at least 25 percent lower than the weight average molecular weight of the cellulose ether used as a starting material for producing the cellulose ether acetate succinate.

11. The process of any one of claims 1 to 10 wherein the molar ratio [succinic anhydride / anhydroglucose units of the cellulose ether] is from 0.2 / 1 to 1.0 / 1.

12. The process of any one of claims 1 to 11, wherein the amount of depolymerizing agent is from 0.03 to 1 mole per mole of anhydroglucose units of the cellulose ether.

**13.** The process of any one of claims 1 to 12 wherein the esterification reaction is conducted in the presence of from 200 to 1000 weight parts of an aliphatic carboxylic acid per 100 weight parts of cellulose ether.

**14.** The process of any one of claims 1 to 13 wherein the esterification reaction is conducted in the presence of an alkali metal carboxylate.

**15.** The process of any one of claims 1 to 14 wherein the cellulose ether has a viscosity of from 1000 mPa·s to 7,000 mPa·s, measured as a 2 weight-% solution in water at 20 °C, the amount of depolymerizing agent is from 0.03 to 0.30 mole per mole of anhydroglucose units of the cellulose ether, and the depolymerizing agent is an oxidizing agent different from oxygen.

**Patentansprüche**

**1.** Verfahren zur Herstellung eines Celluloseetheracetatsuccinats, umfassend den Schritt des Umsetzens eines Celluloseethers mit Essigsäureanhydrid und Bernsteinsäureanhydrid in Gegenwart einer aliphatischen Carbonsäure und mit einem Depolymerisierungsmittel, wobei der Celluloseether eine Anfangsviskosität von wenigstens 50 mPa·s, gemessen als eine 2-gew.%ige Lösung in Wasser bei 20°C, aufweist und das Depolymerisierungsmittel eine anorganische Säure oder ein Oxidationsmittel ist, das sich von Sauerstoff unterscheidet, und die Reaktion des Celluloseethers mit Essigsäureanhydrid, Bernsteinsäureanhydrid und dem Depolymerisierungsmittel gleichzeitig oder aufeinanderfolgend in einem Reaktionsgefäß durchgeführt wird.

**2.** Verfahren nach Anspruch 1, wobei der Celluloseether eine Viskosität von mindestens 500 mPa·s, gemessen als eine 2-gew.%ige Lösung in Wasser bei 20°C, aufweist.

**3.** Verfahren nach Anspruch 1, wobei der Celluloseether eine Viskosität von mindestens 1.000 mPa·s, gemessen als eine 2-gew.%ige Lösung in Wasser bei 20°C, aufweist.

**4.** Verfahren nach einem der Ansprüche 1 bis 3, wobei eine Reaktionsmischung, die den Celluloseether, Essigsäureanhydrid, Bernsteinsäureanhydrid und das Depolymerisierungsmittel enthält, hergestellt wird und die Reaktionsmischung nachfolgend erwärmt wird, um die Veresterungsreaktion durchzuführen.

**5.** Verfahren nach einem der Ansprüche 1 bis 4, wobei die Menge des Depolymerisierungsmittels mindestens 0,03 mol pro mol Anhydroglucoseeinheiten des Celluloseethers beträgt.

**6.** Verfahren nach einem der Ansprüche 1 bis 5, wobei das Depolymerisierungsmittel ausgewählt ist aus der Gruppe bestehend aus Alkalimetallchloriten, Alkalimetallchloraten, Alkalimetallperchloraten, Alkalimetallperiodaten, Alkalimetallhypobromiten, Alkalimetallhypochloriten, Alkalimetallhypoioditen, Alkalimetallperoxiden und Wasserstoffperoxid.

**7.** Verfahren nach Anspruch 6, wobei das Depolymerisierungsmittel Kaliumchlorat oder Natriumchlorat ist.

**8.** Verfahren nach einem der Ansprüche 1 bis 7, wobei der Celluloseether Alkylcellulose, Hydroxyalkylcellulose oder Hydroxyalkylalkylcellulose ist.

**9.** Verfahren nach Anspruch 8, wobei Hydroxypropylmethylcellulose mit Bernsteinsäureanhydrid und Essigsäureanhydrid verestert wird, um Hydroxypropylmethylcelluloseacetatsuccinat herzustellen.

**10.** Verfahren nach einem der Ansprüche 1 bis 9, wobei das gewichtsmittlere Molekulargewicht des Celluloseetheracetatsuccinats mindestens 25 % niedriger als das gewichtsmittlere Molekulargewicht des Celluloseethers ist, der als Ausgangsmaterial für die Herstellung des Celluloseetheracetatsuccinats verwendet wird.

**11.** Verfahren nach einem der Ansprüche 1 bis 10, wobei das Molverhältnis [Bernsteinsäureanhydrid/Anhydroglucoseeinheiten des Celluloseethers] von 0,2 / 1 bis 1,0 / 1 reicht.

**12.** Verfahren nach einem der Ansprüche 1 bis 11, wobei die Menge des Depolymerisierungsmittels von 0,03 bis 1 mol pro mol Anhydroglucoseeinheiten des Celluloseethers reicht.

**13.** Verfahren nach einem der Ansprüche 1 bis 12, wobei die Veresterungsreaktion in Gegenwart von 200 bis 1.000 Gewichtsteilen einer aliphatischen Carbonsäure pro 100 Gewichtsteile Celluloseether durchgeführt wird.

**14.** Verfahren nach einem der Ansprüche 1 bis 13, wobei die Veresterungsreaktion in Gegenwart eines Alkalimetallcarboxylats durchgeführt wird.

**15.** Verfahren nach einem der Ansprüche 1 bis 14, wobei der Celluloseether eine Viskosität von 1.000 mPas bis 7.000 mPa·s, gemessen als eine 2-gew.%ige Lösung in Wasser bei 20°C, aufweist, die Menge an Depolymerisierungsmittel von 0,03 bis 0,30 mol pro mol Anhydroglucoseeinheiten des Celluloseethers reicht und das Depolymerisierungsmittel ein Oxidationsmittel ist, das sich von Sauerstoff unterscheidet.

**Revendications**

**1.** Un procédé pour produire un acétate-succinate d'éther de cellulose comprenant l'étape consistant à faire réagir un éther de cellulose avec de l'anhydride acétique et de l'anhydride succinique en présence d'un acide carboxylique aliphatique et avec un agent de dépolymérisation, dans lequel l'éther de cellulose a une viscosité initiale d'au moins 50 mPa•s, mesurée en tant que solution à 2 % en poids dans de l'eau à 20 °C, l'agent de dépolymérisation est un acide inorganique ou un agent d'oxydation différent de l'oxygène et la réaction de l'éther de cellulose avec l'anhydride acétique, l'anhydride succinique et l'agent de dépolymérisation est effectuée simultanément ou en séquence dans un dispositif de réaction.

**2.** Le procédé de la revendication 1 dans lequel l'éther de cellulose a une viscosité d'au moins 500 mPa•s, mesurée en tant que solution à 2 % en poids dans de l'eau à 20 °C.

**3.** Le procédé de la revendication 1 dans lequel l'éther de cellulose a une viscosité d'au moins 1 000 mPa•s, mesurée en tant que solution à 2 % en poids dans de l'eau à 20 °C.

**4.** Le procédé de n'importe laquelle des revendications 1 à 3 dans lequel un mélange réactionnel comprenant l'éther de cellulose, l'anhydride acétique, l'anhydride succinique et l'agent de dépolymérisation est préparé et le mélange réactionnel est subséquemment chauffé afin d'effectuer la réaction d'estérification.

**5.** Le procédé de n'importe laquelle des revendications 1 à 4 dans lequel la quantité de l'agent de dépolymérisation est d'au moins 0,03 mole par mole d'unités anhydroglucose de l'éther de cellulose.

**6.** Le procédé de n'importe laquelle des revendications 1 à 5 dans lequel l'agent de dépolymérisation est sélectionné dans le groupe constitué de chlorites de métal alcalin, de chlorates de métal alcalin, de perchlorates de métal alcalin, de periodates de métal alcalin, d'hypobromites de métal alcalin, d'hypochlorites de métal alcalin, d'hypoiodites de métal alcalin, de peroxydes de métal alcalin, et de peroxyde d'hydrogène.

**7.** Le procédé de la revendication 6 dans lequel l'agent de dépolymérisation est le chlorate de potassium ou le chlorate de sodium.

**8.** Le procédé de n'importe laquelle des revendications 1 à 7 dans lequel l'éther de cellulose est une alkyl cellulose, une hydroxyalkylcellulose ou une hydroxyalkylalkylcellulose.

**9.** Le procédé de la revendication 8 dans lequel l'hydroxypropyl méthylcellulose est estérifiée avec de l'anhydride succinique et de l'anhydride acétique pour produire de l'acétate-succinate d'hydroxypropyl méthyl cellulose.

**10.** Le procédé de n'importe laquelle des revendications 1 à 9 dans lequel la masse moléculaire moyenne en poids de l'acétate-succinate d'éther de cellulose est d'au moins 25 pour cent plus faible que la masse moléculaire moyenne en poids de l'éther de cellulose utilisé comme matériau de départ pour produire l'acétate-succinate d'éther de cellulose.

**11.** Le procédé de n'importe laquelle des revendications 1 à 10 dans lequel le rapport molaire [anhydride succinique/unités anhydroglucose de l'éther de cellulose] va de 0,2/1 à 1,0/1.

**12.** Le procédé de n'importe laquelle des revendications 1 à 11, dans lequel la quantité d'agent de dépolymérisation va de 0,03 à 1 mole par mole d'unités anhydroglucose de l'éther de cellulose.

13. Le procédé de n'importe laquelle des revendications 1 à 12 dans lequel la réaction d'estérification est effectuée en présence de 200 à 1 000 parties en poids d'un acide carboxylique aliphatique pour 100 parties en poids d'éther de cellulose.

14. Le procédé de n'importe laquelle des revendications 1 à 13 dans lequel la réaction d'estérification est effectuée en présence d'un carboxylate de métal alcalin.

15. Le procédé de n'importe laquelle des revendications 1 à 14 dans lequel l'éther de cellulose a une viscosité allant de 1 000 mPa•s à 7 000 mPa•s, mesurée en tant que solution à 2 % en poids dans de l'eau à 20 °C, la quantité d'agent de dépolymérisation va de 0,03 à 0,30 mole par mole d'unités anhydroglucose de l'éther de cellulose, et l'agent de dépolymérisation est un agent d'oxydation différent de l'oxygène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4226981 A
- US 5776501 A
- WO 2005115330 A
- WO 2011159626 A
- EP 1141029 A
- EP 210917 A
- EP 1423433 A
- US 4316982 A
- US 3629237 A
- EP 0219426 A
- US 13030394 W
- WO 2013148154 A

### Non-patent literature cited in the description

- **KEARY, C.M.** *Carbohydrate Polymers,* 2001, vol. 45, 293-303
- Hypromellose. United States Pharmacopeia (USP, vol. 35, 423-424, 3467-3469
- **G. BARTELMUS ; R. KETTERER, Z.** *Anal. Chem.,* 1977, vol. 286, 161-190
- Hypromellose. European Pharmacopeia Ph Eur, 2008, vol. 6, 2819
- Hypromellose Acetate Succinate. United States Pharmacopia and National Formulary, NF, vol. 29, 1548-1550
- Hypromellose. United States Pharmacopeia and National Formulary, USP, vol. 35, 3467-3469
- *Journal of Pharmaceutical and Biomedical Analysis,* 2011, vol. 56, 743-748
- Hypromellose Acetate Succinate. *United States Pharmacopia and National Formulary, NF,* vol. 29, 1548-1550
- Hypromellose Acetate Succinate. United States Pharmacopia and National Formulary, NF, vol. 29
- *Journal of Pharmaceutical and Biomedical Analysis,* 2011, vol. 56, 743
- **CHEN, R. et al.** *Journal of Pharmaceutical and Biomedical Analysis,* 2011, vol. 56, 743-748